# EUROPEAN PATENT SPECIFICATION

(11) **EP 2 767 951 B1**
(45) Date of publication and mention of the grant of the patent: **04.09.2019**
(21) Application number: 12839520.9
(22) Date of filing: 05.10.2012
(51) Int. Cl.: G16H 10/60, G06F 21/00

(54) **INFORMATION PROCESSING DEVICE, METHOD AND PROGRAM**
VORRICHTUNG, VERFAHREN UND PROGRAMM ZUR INFORMATIONSVERARBEITUNG
DISPOSITIF, PROCÉDÉ ET PROGRAMME DE TRAITEMENT DE DONNÉES

(30) Priority: 13.10.2011 JP 2011225880; 01.02.2012 JP 2012020158
(43) Date of publication of application: 20.08.2014
(73) Proprietor: Sony Corporation, Tokyo 108-0075 (JP)
(72) Inventor: ARAYA Shinsuke, Tokyo 108-0075 (JP); FUKUSHI Gakuho, Tokyo 108-0075 (JP)
(74) Representative: D Young & Co LLP
(86) International application number: PCT/JP2012/075920
(87) International publication number: WO 2013/054749

(56) References cited:
- WO-A2-00/51036
- JP-A- 2001 155 100
- JP-A- 2002 117 142
- JP-A- 2008 108 021
- JP-A- 2010 026 899
- US-B1- 6 654 724
- M. RAJARAJAN ET AL: "Patient Privacy Protection Using Anonymous Access Control Techniques", METHODS OF INFORMATION IN MEDICINE, 1 January 2008 (2008-01-01), XP055188628, ISSN: 0026-1270, DOI: 10.3414/ME9116

## Description

### Technical Field

The present technology relates to an information processing apparatus and method, and a program, and in particular, relates to an information processing apparatus and method capable of improving security and a program.

### Background Art

While prescriptions written by physicians and drug history handbooks issued by pharmacies are currently operated by means of paper media, the construction of a mechanism to electronically share and manage health data called EHR (Electric Health Record) or the like is demanded from the viewpoint of convenience and improvement of efficiency. Currently, for example, sharing of electronic medical records, digitization of prescriptions, and digitization of drug history handbooks are promoted.

For example, as a technology concerning the drug history handbook, a technology to record information about drug names, dosage methods and the like in advance and to create and print drug instructions based on the information is proposed (see, for example, Patent Literature 1). According to the technology, storage information is provided in a portion of drug instructions so that information about drugs can also be stored in a handbook possessed by a patient.

### Citation List

### Patent Literature

Patent Literature 1: JP H11-028877A

US 6654724 describes a system for processing data including patient identity information and pharmaceutical information. The system permits analysis of the pharmaceutical information to occur while maintaining the confidentiality of the patient identity information.

### Summary of Invention

### Technical Problem

In a system sharing and managing health data such as digitized drug history handbooks and prescriptions as described above, each user's personal information is handled and security improvement is desired.

The present technology is developed in view of such circumstances and intended to achieve improvement of security.

### Solution to Problem

According to the invention, there is provided an information processing apparatus including a receiving unit that receives personal identification information corresponding to a user for which personal information identifying the user is stored in a local system and system identification information to identify the local system, wherein the local system is configured to store personal information for a plurality of users and to associate the personal information identifying the user with the user corresponding to the personal identification information based on system internal user identification information in order to identify the user, a recording unit that associates and records the personal identification information, the system identification information, the system internal user identification information used in the local system to identify the user, and shared data about the user, wherein the shared data is data on health of the user that is shared by the local system and the information processing apparatus; a data search unit that searches for the shared data associated with the received personal identification information, a user search unit that searches for the system internal user identification information associated with the received personal identification information and the received system identification information, and a transmitting unit that transmits the shared data and the system internal user identification information that are obtained in a search.

The shared data may be data on health of the user.

The receiving unit may further include an update unit that receives the personal identification information, the system identification information, the system internal user identification information, and the shared data which is new and associates the personal identification information, the system identification information, the system internal user identification information, and the new shared data that are received, to record the information and the data in the recording unit.

The system identification information may be a hash value obtained by hashing information determining the local system.

According to the invention, there is provided an information processing method or a program including receiving personal identification information corresponding to a user for which personal information identifying the user is stored in a local system and system identification information to identify the local system, wherein the local system stores personal information for a plurality of users and associates the personal information identifying the user with the user corresponding to the personal identification information based on system internal user identification information in order to identify the user; searching for shared data associated with the received personal identification information in the personal identification information, the system identification information, system internal user identification information used in the local system to identify the user, and the shared data about the user that are recorded in a recording unit by being associated with each other, searching for the system internal user identification information associated with the received personal identification information and the received system identification information in the personal identification information, the system identification information, the system internal user identification information, and the shared data that are recorded in the recording unit by being associated with each other, and transmitting the shared data and the system internal user identification information that are obtained in a search.

According to the invention, personal identification information to identify a user and system identification information to identify a local system is to be received, shared data associated with the received personal identification information is to be searched for, in the personal identification information, the system identification information, system internal user identification information used in the local system to identify the user, and the shared data about the user that are recorded in a recording unit by being associated with each other, the system internal user identification information associated with the received personal identification information and the received system identification information is to be searched for, in the personal identification information, the system identification information, the system internal user identification information, and the shared data that are recorded in the recording unit by being associated with each other, and the shared data and the system internal user identification information that are obtained in a search is to be transmitted.

According to the invention, there is provided an information processing apparatus including an acquisition unit that acquires personal identification information corresponding to a user for which personal information identifying the user is stored by a local system including the information processing apparatus, a transmitting unit that transmits system identification information identifying the local system including the information processing apparatus, and the personal identification information to another information processing apparatus that associates and records the personal identification information, the system identification information, system internal user identification information used in the local system to identify the user, and shared data about the user, and a receiving unit that receives, from the other information processing apparatus, the shared data associated with the transmitted personal identification information and the system internal user identification information associated with the personal identification information and the system identification information that are transmitted, wherein the information processing apparatus is configured to store personal information for a plurality of users and to associate the personal information identifying the user with the user corresponding to the personal identification information based on the system internal user identification information in order to identify the user.

The shared data may be data on health of the user.

The system identification information may be a hash value obtained by hashing information determining the local system.

The local system may include a recording unit that associates and records the system identification information, the system internal user identification information, and the shared data.

According to the invention, there is provided an information processing method including acquiring personal identification information corresponding to a user for which personal information identifying the user is stored by a local system including the information processing apparatus, transmitting system identification information identifying a local system including the information processing apparatus and the personal identification information to another information processing apparatus that associates and records the personal identification information, the system identification information, system internal user identification information used in the local system to identify the user, and shared data about the user, and receiving, from the other information processing apparatus, the shared data associated with the transmitted personal identification information and the system internal user identification information associated with the personal identification information and the system identification information that are transmitted; and storing personal information for a plurality of users and associating the personal information identifying the user with the user corresponding to the personal identification information based on the system internal user identification information in order to identify the user.

According to the invention, personal identification information identifying a user is to be received, system identification information identifying a local system including the information processing apparatus and the personal identification information is to be transmitted to another information processing apparatus that associates and records the personal identification information, the system identification information, system internal user identification information used in the local system to identify the user, and shared data about the user, and the shared data associated with the transmitted personal identification information and the system internal user identification information associated with the personal identification information and the system identification information that are transmitted are to be received from the other information processing apparatus.

### Advantageous Effects of Invention

According to a first aspect and a second aspect of the present technology, improvement of security can be achieved.

### Brief Description of Drawings

FIG. 1 is a diagram showing a configuration example according to an example of an information processing system to which the present technology is applied.
FIG. 2 is a diagram showing an example of personal/drug history information.
FIG. 3 is a diagram showing an example of user drug history information.
FIG. 4 is a flow chart illustrating a registration request process.
FIG. 5 is a flow chart illustrating a registration process.
FIG. 6 is a flow chart illustrating a reading process and a providing process.
FIG. 7 is a diagram showing a configuration example of a computer.
FIG. 8 is a diagram illustrating an example of recording locations of personal information in the information processing system.
FIG. 9 is a diagram illustrating an example of recording locations of personal information in the information processing system.
FIG. 10 is a diagram illustrating an example of recording locations of personal information in the information processing system.

### Description of Embodiment

An example to which the present technology is applied will be described below with reference to the drawings.

### <First example>

### [Configuration example of the information processing system]

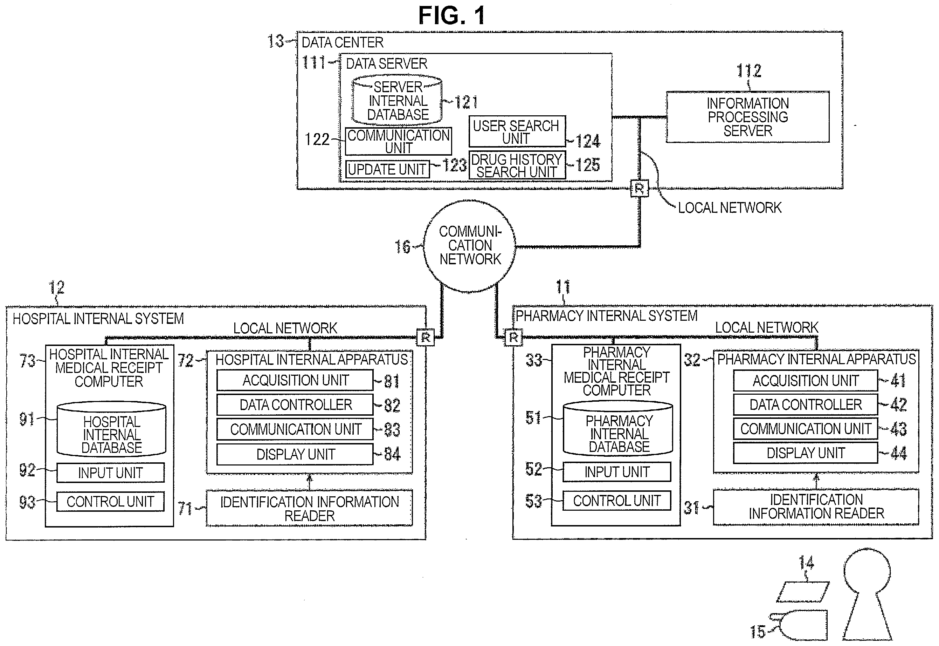

FIG. 1 is a diagram showing a configuration example of an information processing system to which the present technology is applied.

The information processing system includes a pharmacy internal system 11, a hospital internal system 12, a data center 13, an IC (Integrated Circuit) card 14 possessed by a user as a patient, and a mobile terminal apparatus 15. The pharmacy internal system 11 to the data center 13 are mutually connected via a communication network 16 formed from a wire or wireless network such as the Internet.

The pharmacy internal system 11 is provided in a pharmacy where the patient purchases prescribed drugs and includes an identification information reader 31, a pharmacy internal apparatus 32, and a pharmacy internal medical receipt computer 33. The pharmacy internal apparatus 32 and the pharmacy internal medical receipt computer 33 are mutually connected via a local network and the local network is connected to the communication network 16 via a router or the like.

The identification information reader 31 wirelessly communicates with the IC card 14 of a patient or the mobile terminal apparatus 15 to read a personal identification ID as an ID specific to each patient from the IC card 14 or the mobile terminal apparatus 15 and supplies the personal identification ID to the pharmacy internal apparatus 32.

The personal identification ID is identification information capable of uniquely identifying the user, but is regarded as information that is generally unable to (or hard to) determine each individual user identified by the personal identification ID only by the personal identification ID being acquired by others. For example, personal information such as the name, address, and telephone number allows to determine the user almost directly from such information. However, if an ID made of numbers or symbols is attached to each user as a personal identification ID, it is difficult to determine the user of the personal identification ID from the personal identification ID as long as the correspondence between the personal identification ID and the user is not known.

The medium to serve as a motivation for acquiring the personal identification ID is not limited to an IC card in which the personal identification ID is directly recorded and includes any specific medium that cannot be rewritten or replaced. When, for example, specific information is acquired from living body information as a personal identification ID, the medium to acquire the personal identification ID from is a portion or the whole of a human body. The description below continues by assuming that the personal identification ID is a specific ID held by a non-contact IC provided in the mobile terminal apparatus 15 possessed by the user. For example, the mobile terminal apparatus 15 is a mobile phone.

The pharmacy internal apparatus 32 is, for example, a computer or the like and performs various processes in accordance with the personal identification ID supplied by the identification information reader 31 or an input operation by a pharmacist or the like.

The pharmacy internal apparatus 32 includes an acquisition unit 41, a data controller 42, a communication unit 43, and a display unit 44. The acquisition unit 41 acquires a personal identification ID from the identification information reader 31. The data controller 42 exchanges various kinds of data with the pharmacy internal medical receipt computer 33 via a local network or causes the display unit 44 to display data.

The communication unit 43 transmits/receives information to/from other apparatuses via a local network or the communication network 16. The display unit 44 displays various kinds of data or images.

The pharmacy internal medical receipt computer 33 includes a pharmacy internal database 51, an input unit 52, and a control unit 53 and updates information recorded in the pharmacy internal database 51 in accordance with an input operation of a pharmacist or the like.

The input unit 52 is, for example, a mouse and supplies information in accordance with an input operation of the user to the control unit 53. The control unit 53 controls the whole pharmacy internal medical receipt computer 33 in accordance with information supplied by the input unit 52. In the pharmacy internal database 51, personal/drug history information including personal information of a user as a patient and health data as data on user's health is recorded for each user.

In this example, health data is data on user's drug history (hereinafter, called drug history data). For example, drug history data contained in personal/drug history information includes dispensing data including information about drugs dispensed for the user in a pharmacy or the like and information about a prescription for the drugs. Health data managed by the pharmacy internal database 51 is not limited to drug history data and may be any data on user's health, but in the description that follows, it is assumed that health data is drug history data.

Drug history data (dispensing data) recorded in the pharmacy internal database 51 is transmitted and recorded in the data center 13 if necessary and shared among a plurality of the pharmacy internal systems 11 or the hospital internal systems 12.

Incidentally, the identification information reader 31, the pharmacy internal apparatus 32, and the pharmacy internal medical receipt computer 33 may be configured by one apparatus.

The hospital internal system 12 is provided in a hospital to which the user goes as a patient and includes an identification information reader 71, a hospital internal apparatus 72, and a hospital internal medical receipt computer 73. The hospital internal apparatus 72 and the hospital internal medical receipt computer 73 are mutually connected via a local network and the local network is connected to the communication network 16 via a router or the like.

The identification information reader 71, the hospital internal apparatus 72, and the hospital internal medical receipt computer 73 constituting the hospital internal system 12 correspond to the identification information reader 31, the pharmacy internal apparatus 32, and a pharmacy internal medical receipt computer 33 of the pharmacy internal system 11 respectively and the configuration and operation thereof are similar and thus, a detailed description thereof is omitted.

The identification information reader 71 is connected to the hospital internal apparatus 72 in the hospital internal system 12 and the hospital internal apparatus 72 is provided with and includes an acquisition unit 81, a data controller 82, a communication unit 83, and a display unit 84. The acquisition unit 81 to the display unit 84 are similar to the acquisition unit 41 to the display unit 44 of the pharmacy internal apparatus 32 respectively and thus, a detailed description thereof is omitted.

The hospital internal medical receipt computer 73 is provided with a hospital internal database 91, an input unit 92, and a control unit 93 and the hospital internal database 91 to the control unit 93 correspond to the pharmacy internal database 51 to the control unit 53 of the pharmacy internal medical receipt computer 33 respectively.

In the hospital internal database 91, however, personal/diagnosis information including personal information of a user as a patient and drug history data as health data of data on user's health is recorded for each user. For example, drug history data contained in the personal/diagnosis information contains diagnosis data including information about prescriptions issued to the user by hospitals or the like.

Drug history data (diagnosis data) recorded in the hospital internal database 91 is transmitted and recorded in the data center 13 if necessary and shared among a plurality of the pharmacy internal systems 11 or the hospital internal systems 12.

Incidentally, the identification information reader 71, the hospital internal apparatus 72, and the hospital internal medical receipt computer 73 may be configured by one apparatus.

The data center 13 includes a data server 111 and an information processing server 112. The data server 111 and the information processing server 112 are mutually connected via a local network and the local network is connected to the communication network 16 via a router or the like.

The data server 111 manages drug history data supplied by the pharmacy internal system 11 or the hospital internal system 12 and also provides recorded drug history data or the like in response to a request from the pharmacy internal system 11 or the hospital internal system 12. The data server 111 includes a server internal database 121, a communication unit 122, an update unit 123, a user search unit 124, and a drug history search unit 125.

The server internal database 121 records user drug history information for each registered user. In the user drug history information, information to determine the pharmacy internal system 11 and the like, identification information of users in a local system such as the pharmacy internal system 11, and drug history data of users supplied by the pharmacy internal system 11 are contained.

The communication unit 122 transmits/receives various kinds of data such as drug history data by communicating with the pharmacy internal system 11 or the hospital internal system 12 via the communication network 16. When drug history data or the like is supplied by the pharmacy internal system 11 or the hospital internal system 12, the update unit 123 updates user drug history information based on the drug history data.

The user search unit 124 searches for information to determine the desired user from drug history information recorded in the server internal database 121, that is, identification information of the user used in a local system such as the pharmacy internal system 11. The drug history search unit 125 searches for drug history data of the desired user from drug history information recorded in the server internal database 121.

The information processing server 112 accesses the server internal database 121 of the data server 111 if necessary to process user drug history information or to perform a notification process to each user based on the user drug history information.

In the example of FIG. 1, the one pharmacy internal system 11 and the one hospital internal system 12 are included in the information processing system, but actually, a plurality of the pharmacy internal systems 11 and a plurality of the hospital internal systems 12 are included in the information processing system.

### [Pharmacy internal database]

Next, a concrete example of personal/drug history information recorded in the pharmacy internal database 51 of the pharmacy internal system 11 in FIG. 1 will be described.

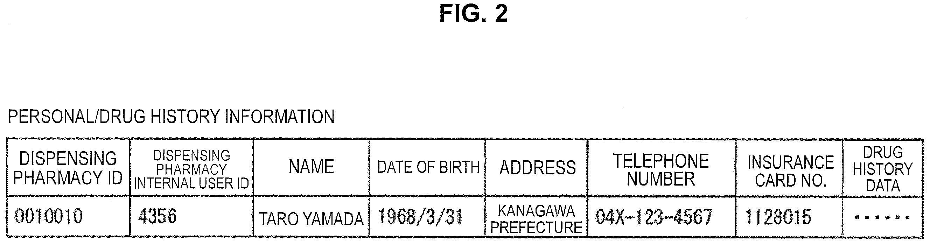

For example, the personal/drug history information shown in FIG. 2 is recorded for each user as a patient in the pharmacy internal database 51. In the example of FIG. 2, the personal/drug history information contains "Dispensing pharmacy ID", "Dispensing pharmacy internal user ID", "Name", "Date of birth", "Address", "Telephone number", "Insurance card No.", and "Drug history data".

"Dispensing pharmacy ID" is information that can be identified by each apparatus constituting the information processing system shown in FIG. 1 to determine the pharmacy internal system 11, that is, the pharmacy. For example, the medical institution code authorized to treat patients with health insurance coverage used when each institution such as a pharmacy makes a payment request to an insurer is set as the dispensing pharmacy ID.

"Dispensing pharmacy internal user ID" is information to identify the user used only in the pharmacy internal system 11, that is, the local system including the identification information reader 31 to the pharmacy internal medical receipt computer 33. For example, the user No. registered with the pharmacy internal medical receipt computer 33 constituting the pharmacy internal system 11 is set as the dispensing pharmacy internal user ID.

Therefore, only each apparatus constituting the pharmacy internal system 11 such as the pharmacy internal apparatus 32 can determine the user indicated by the dispensing pharmacy internal user ID based on the dispensing pharmacy internal user ID. The dispensing pharmacy internal user ID is information that does not allow to directly determine the user only by being acquired by others.

"Name", "Date of birth", "Address", "Telephone number", and "Insurance card No." contained in personal/drug history information are the name, date of birth, address, telephone number, and insurance card No. of the user determined by "Dispensing pharmacy internal user ID". "Name" to "Insurance card No." are so-called user's personal information with which others can directly determine the user.

"Drug history data" contained in personal/drug history information is drug history data of the user determined by "Dispensing pharmacy internal user ID" and the drug history data contains dispensing data input through the pharmacy internal system 11. That is, drug history data recorded in the pharmacy internal database 51 of each pharmacy (pharmacy internal system 11) contains only information input in the pharmacy and does not contain information about the drug history input in other pharmacies or hospitals (other medical institutions and the like).

More specifically, drug history data contains, for example, the dispensed drug ID determining the dispensed drug, type of the dispensed drug such as oral administration, dispensing date/time, number of days of prescription of the drug, frequency of using the drug, dosage of the drug, and information about side effects as dispensing data.

Thus, the personal/drug history information contains the dispensing pharmacy ID to determine the pharmacy, dispensing pharmacy internal user ID to identify the user in the pharmacy, personal information of the user such as the name, and drug history data input in the pharmacy.

In each of the pharmacy internal systems 11 and the hospital internal systems 12 constituting the information processing system in FIG. 1, personal/drug history information or personal/diagnosis information containing drug history data input in each system is managed by the pharmacy internal database 51 or the hospital internal database 91 for each user.

Drug history data contained in personal/diagnosis information managed by the hospital internal database 91 contains information (diagnosis data) about prescriptions issued to the users in the hospital. More specifically, the drug history data contains, for example, the date/time of issue of a prescription, whether to allow changes to generic drugs, prescribed drug ID to determine the prescribed drug, type of the prescribed drug such as oral administration, number of days of prescription of the drug, frequency of using the drug, dosage of the drug, and information about side effects.

Personal/diagnosis information managed by the hospital internal database 91 contains, instead of "Dispensing pharmacy ID" and "Dispensing pharmacy internal user ID" in FIG. 2, "Hospital ID" and "Hospital internal user ID". "Hospital ID" is information corresponding to "Dispensing pharmacy ID" and is information to determine the hospital internal system 12. "Hospital internal user ID" is information corresponding to "Dispensing pharmacy internal user ID" and is identification information of the user used only inside the hospital internal system 12.

### [Server internal database]

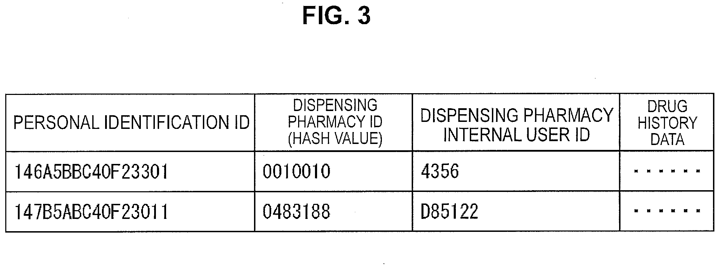

Further, for example, user drug history information shown in FIG. 3 is recorded in the server internal database 121 of the data server 111 in FIG. 1. In the example of FIG. 3, each piece of user drug history information contains "Personal identification ID", "Dispensing pharmacy ID", "Dispensing pharmacy internal user ID", and "Drug history data".

"Personal identification ID" contained in the user drug history information is a personal identification ID recorded in the mobile terminal apparatus 15 in FIG. 1. The personal identification ID is used, as described above, as information to determine the user possessing the mobile terminal apparatus 15.

"Dispensing pharmacy ID" is a dispensing pharmacy ID to determine the pharmacy internal system 11. More specifically, "Dispensing pharmacy ID" contained in the user drug history information is a hash value obtained by hashing the dispensing pharmacy ID.

"Dispensing pharmacy internal user ID" is a dispensing pharmacy internal user ID used in the pharmacy internal system 11 to identify the user and "Drug history data" is drug history data determined by the dispensing pharmacy ID and input in the pharmacy internal system 11.

More specifically, in user drug history information containing drug history information concerning the hospital internal system 12, instead of "Dispensing pharmacy ID" and "Dispensing pharmacy internal user ID", "Hospital ID" and "Hospital internal user ID" are used.

### [User registration]

If drug history data input in a medical institution or the like is recorded in the server internal database 121 by associating with personal identification ID or the like as user drug history data, drug history data contained in such user drug history data can also be used by other medical institutions and the like (local systems).

To share user drug history data, it is necessary to first register the user. That is, it is necessary to associate the personal identification ID to determine the user with the dispensing pharmacy internal user ID or the like used in each of the pharmacy internal systems 11 or the like.

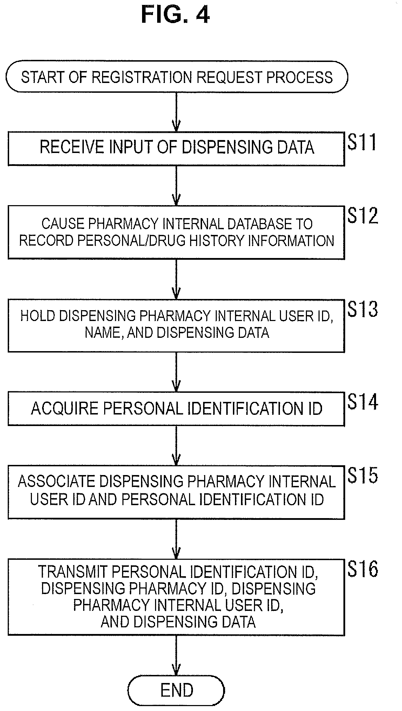

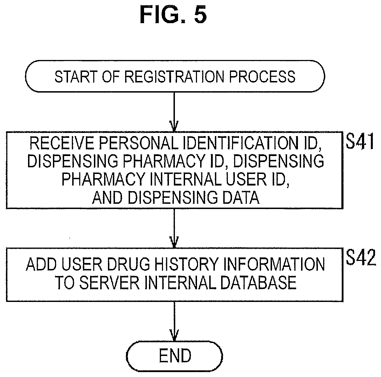

Hereinafter, a registration request process in which the pharmacy internal system 11 requests user registration and a registration process in which the data server 111 registers the user in response to the request will be described with reference to the flow charts in FIGS. 4 and 5.

FIG. 4 is a flow chart illustrating a registration request process by the pharmacy internal system 11.

When a new user visits a pharmacy provided with the pharmacy internal system 11 and presents a prescription, a pharmacist or the like in the pharmacy operates the input unit 52 of the pharmacy internal medical receipt computer 33 based on the presented prescription to start input of the user's dispensing data.

Then, in step S11, the control unit 53 receives input of the dispensing data and acquires information in accordance with the operation of the input unit 52 by the pharmacist or the like as the dispensing data.

When the user visits a pharmacy provided with the pharmacy internal system 11 for the first time, personal information and other information about the user are not registered with (recorded in) the pharmacy internal database 51 and thus, the pharmacist or the like operates the input unit 52 to input the name, date of birth and the like of the user.

When, for example, the personal/drug history information shown in FIG. 2 is recorded in the pharmacy internal database 51, the name, date of birth, address, telephone number, and insurance card No. of the user are input by the pharmacist or the like and if necessary, the dispensing pharmacy internal user ID of the user is attached. When the above information is input, the control unit 53 generates drug history data from dispensing data acquired from the input unit 52 and also generates personal/drug history information from information of the input name and the like of the user, the generated drug history data, and the dispensing pharmacy internal user ID attached to the user.

If the user has visited the pharmacy and personal/drug history information of the user is already recorded in the pharmacy internal database 51, no personal/drug history information is generated.

In step S12, the control unit 53 causes the pharmacy internal database 51 to record the generated personal/drug history information by supplying the information thereto and also supplies the dispensing pharmacy internal user ID, user's name, and dispensing data (drug history data) acquired in the process of step S11 to the pharmacy internal apparatus 32 via a local network.

In step S13, the data controller 42 of the pharmacy internal apparatus 32 temporarily holds the dispensing pharmacy internal user ID, user's name, and dispensing data supplied by the control unit 53.

When inputting dispensing data and personal information of the user is finished, the pharmacist or the like dispenses a prescribed drug and administers the drug to the user if necessary. Further, when the user visits the pharmacy for the first time, the pharmacist or the like checks to see whether the user desires new registration of an electronic drug history handbook. That is, the pharmacist or the like checks to see whether user registration with the data center 13 is desired.

If the user desires new registration of an electronic drug history handbook, the pharmacist or the like operates the pharmacy internal apparatus 32 to perform a process of new registration. The data controller 42 of the pharmacy internal apparatus 32 causes the display unit 44 to display the temporarily held dispensing pharmacy internal user ID, user's name, and dispensing data by supplying the data thereto in accordance with an operation by the pharmacist or the like.

The pharmacist or the like and the user confirm information such as the name displayed in the display unit 44 and then, the user holds the mobile terminal apparatus 15 possessed by the user over the identification information reader 31. Then, the identification information reader 31 wirelessly communicates with the mobile terminal apparatus 15 to receive the personal identification ID from the mobile terminal apparatus 15.

In step S14, the acquisition unit 41 acquires the user's personal identification ID from the identification information reader 31 and supplies the ID to the data controller 42.

In step S15, the data controller 42 associates the temporarily held dispensing pharmacy internal user ID of the user and the personal identification ID supplied by the acquisition unit 41.

The data controller 42 acquires the dispensing pharmacy ID of the pharmacy internal system 11 from the pharmacy internal medical receipt computer 33 if necessary and hashes the dispensing pharmacy ID. Then, the data controller 42 supplies the hash value (dispensing pharmacy ID) obtained by a hashing operation and also the personal identification ID, dispensing pharmacy internal user ID, and dispensing data to the communication unit 43.

In step S16, the communication unit 43 transmits the dispensing pharmacy ID (hash value), personal identification ID, dispensing pharmacy internal user ID, and dispensing data supplied by the data controller 42 to the data server 111 via the communication network 16 to complete the registration request process. That is, the communication unit 43 transmits a new registration request of the user containing the dispensing pharmacy ID, personal identification ID, dispensing pharmacy internal user ID, and dispensing data.

When a new registration request is obtained after being transmitted by the pharmacy internal system 11, the data server 111 performs a registration process according to the request. Hereinafter, the registration process by the data server 111 will be described with reference to the flow chart in FIG. 5.

In step S41, the communication unit 122 receives and supplies the dispensing pharmacy ID (hash value), personal identification ID, dispensing pharmacy internal user ID, and dispensing data transmitted by the pharmacy internal system 11 to the update unit 123.

In step S42, the update unit 123 generates user drug history information containing the dispensing pharmacy ID (hash value), personal identification ID, dispensing pharmacy internal user ID, and dispensing data supplied by the communication unit 122 and adds the generated user drug history information to the server internal database 121. That is, the newly generated user drug history information is recorded in the server internal database 121. Accordingly, for example, user drug history information shown in FIG. 3 is newly recorded. When user drug history information is generated, the update unit 123 sets the dispensing data of the user directly as drug history information.

When the newly generated user drug history information is recorded in the server internal database 121, new registration of the user is completed to finish the registration process.

As described above, the pharmacy internal system 11 requests the registration of a user by transmitting the dispensing pharmacy ID determining the pharmacy, personal identification ID determining the user, dispensing pharmacy internal user ID, and dispending data of the user to the data server 111. The data server 111 receives the information transmitted by the pharmacy internal system 11 and generates and registers user drug history information.

For the registration, the pharmacy internal system 11 and the data server 111 exchange the personal identification ID and dispensing pharmacy internal user ID via the communication network 16 as information to determine the user. However, even if such information is viewed by others, it is generally almost impossible to determine the individual user and therefore, leakage of personal information can be prevented and security can thereby be improved.

In addition, the pharmacy internal system 11 and the data server 111 also exchange the dispensing pharmacy ID determining the pharmacy for the registration and the dispensing pharmacy ID is hashed, that is, a has value. Therefore, it is difficult for others to determine the pharmacy from the hash value and therefore, security can be improved.

### [Update/reference of drug history data]

When drug history data of the user is recorded in the data server 111 as described above, each of the pharmacy internal systems 11 and the hospital internal systems 12 can refer to drug history data of the user and update drug history data of the user.

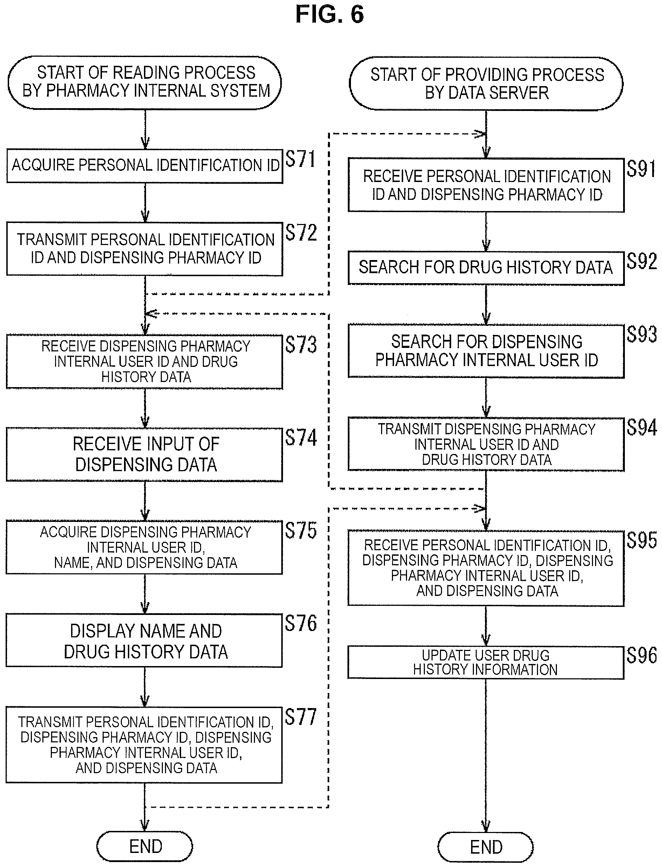

A reading process by the pharmacy internal system 11 and a provisioning process by the data server 111 will be described with reference to the flow chart in FIG. 6.

The user visits a dispensing pharmacy and presents a prescription issued by a hospital and also holds the mobile terminal apparatus 15 possessed by the user over the identification information reader 31.

Then, in step S71, the acquisition unit 41 acquires the personal identification ID. That is, the identification information reader 31 wirelessly communicates with the mobile terminal apparatus 15 to receive the personal identification ID from the mobile terminal apparatus 15. The acquisition unit 41 acquires a personal identification ID from the identification information reader 31 and supplies the ID to the data controller 42.

The data controller 42 acquires the dispensing pharmacy ID from the pharmacy internal medical receipt computer 33 if necessary and hashes the dispensing pharmacy ID. Then, the data controller 42 supplies the hash value (dispensing pharmacy ID) obtained by a hashing operation and personal identification ID to the communication unit 43.

In step S72, the communication unit 43 transmits the personal identification ID and dispensing pharmacy ID (hash value) supplied by the data controller 42 to the data server 111 via the communication network 16 and also request the transmission of the dispensing pharmacy internal user ID and drug history data. That is, a transmission request containing the personal identification ID and dispensing pharmacy ID is transmitted.

Then, in step S91, the communication unit 122 of the data server 111 receives the personal identification ID and dispensing pharmacy ID (hash value) transmitted by the pharmacy internal system 11.

In step S92, the drug history search unit 125 searches for drug history data determined by the personal identification ID received by the communication unit 122 in response to a request from the pharmacy internal system 11.

That is, the drug history search unit 125 searches for user drug history information containing the personal identification ID received by the communication unit 122 from user drug history information recorded in the server internal database 121. Then, the drug history search unit 125 supplies drug history data contained in the user drug history information obtained by the search to the communication unit 122.

In the search for drug history data described above, a search using the personal identification ID as a key is performed. Thus, the drug history data obtained by the search contains not only drug history data input by the pharmacy internal system 11 that have requested the search, but also drug history data input by other pharmacies and hospitals in the past.

In step S93, the user search unit 124 searches for the dispensing pharmacy internal user ID determined by the personal identification ID received by the communication unit 122 and the dispensing pharmacy ID (hash value) in response to a request from the pharmacy internal system 11.

That is, the user search unit 124 searches for user drug history information containing the personal identification ID received by the communication unit 122 and the dispensing pharmacy ID from user drug history information recorded in the server internal database 121. Then, the user search unit 124 supplies the dispensing pharmacy internal user ID contained in the user drug history information obtained by the search to the communication unit 122. Thus, the search for the dispensing pharmacy internal user ID is performed by using the personal identification ID and dispensing pharmacy internal user ID as keys.

In step S94, the communication unit 122 transmits the drug history data supplied by the drug history search unit 125 and the dispensing pharmacy internal user ID supplied by the user search unit 124 to the pharmacy internal system 11 via the communication network 16.

In step S73, the communication unit 43 of the pharmacy internal apparatus 32 receives and supplies the dispensing pharmacy internal user ID and drug history data transmitted by the data server 111 to the data controller 42.

In step S74, the control unit 53 of the pharmacy internal medical receipt computer 33 receives input of dispensing data.

For example, a pharmacist or the like inputs dispensing data based on the prescription presented by the user. More specifically, a pharmacist or the like operates the pharmacy internal medical receipt computer 33 to input dispensing data of a drug newly dispensed this time based on the prescription.

Then, the control unit 53 acquires dispensing data in accordance with an operation by the pharmacist or the like from the input unit 52 and also adds the acquired dispensing data to drug history data contained in personal/drug history information of the user recorded in the pharmacy internal database 51. Accordingly, the drug history data of the user is updated.

The control unit 53 also reads the dispensing pharmacy internal user ID and name of the user from the personal/drug history information of the user recorded in the pharmacy internal database 51 and also supplies the read dispensing pharmacy internal user ID and name and the dispensing data this time to the pharmacy internal apparatus 32. The dispensing data supplied to the pharmacy internal apparatus 32 is dispensing data newly input in the process of step S74.

In step S75, the data controller 42 of the pharmacy internal apparatus 32 acquires the dispensing pharmacy internal user ID, name, and dispensing data from the control unit 53.

Accordingly, the data controller 42 temporarily holds the dispensing pharmacy internal user ID, name, and dispensing data from the pharmacy internal medical receipt computer 33, the dispensing pharmacy internal user ID and drug history data from the data server 111, and the personal identification ID from the identification information reader 31.

Because both of data acquired from the pharmacy internal medical receipt computer 33 and data acquired from the data server 111 contain the dispensing pharmacy internal user ID, the above data is strung together to indicate that each piece of data belongs to the same user based on the dispensing pharmacy internal user ID.

The data controller 42 causes the display unit 44 to display the acquired name, dispensing data, and drug history data of the user by supplying the data thereto.

In step S76, the display unit 44 displays the user's name, dispensing data, and drug history data supplied by the data controller 42.

The pharmacist or the like administers a drug to the user by referring to the past drug history data displayed in the display unit 44 and newly input dispensing data. When the administration is finished, the pharmacist or the like checks to see whether the user desires the dispensing data of the drug prescribed this time to be registered with the data server 111.

If the user desires the registration, the pharmacist or the like operates the pharmacy internal apparatus 32 to instruct the registration of the dispensing data, that is, an update of the data server 111 with the user drug history information.

Then, the data controller 42 hashes the dispensing pharmacy ID in accordance with an operation of the pharmacist or the like and also supplies the hash value (dispensing pharmacy ID) obtained by hashing, personal identification ID, dispensing pharmacy internal user ID, and dispensing data to the communication unit 43. The dispensing data supplied to the communication unit 43 is dispensing data newly input in the process of step S74.

In step S77, the communication unit 43 transmits the dispensing pharmacy ID (hash value), personal identification ID, dispensing pharmacy internal user ID, and dispensing data supplied by the data controller 42 to the data server 111 via the communication network 16 to request an update of the user drug history information. That is, an update request of the user drug history information (drug history information) containing the dispensing pharmacy ID, personal identification ID, dispensing pharmacy internal user ID, and dispensing data is transmitted. When the request is transmitted, the reading process by the pharmacy internal system 11 is finished.

In step S95, the communication unit 122 of the data server 111 receives and supplies the dispensing pharmacy ID (hash value), personal identification ID, dispensing pharmacy internal user ID, and dispensing data transmitted by the pharmacy internal system 11 to the update unit 123.

In step S96, the update unit 123 updates the user drug history information in response to the request from the pharmacy internal system 11 before finishing the providing process.

That is, the update unit 123 generates new user drug history information containing the dispensing pharmacy ID (hash value), personal identification ID, dispensing pharmacy internal user ID, and dispensing data supplied by the communication unit 122 and causes the server internal database 121 to record the generated user drug history information. Accordingly, the user drug history information recorded in the server internal database 121 is updated.

When user drug history information is generated, dispensing data received by the communication unit 122 is set as drug history data contained in user drug history information.

The pharmacy internal system 11 reads, as described above, past drug history data of the user from the data server 111 by using the personal identification ID as a key and also causes the database to record the user drug history information containing the newly generated drug history data.

When drug history data is read or drug history data (user drug history information) is updated, the personal identification ID or dispensing pharmacy internal user ID identifying the user and the hash value of the dispensing pharmacy ID identifying the pharmacy are exchanged between the data server 111 and the pharmacy internal system 11. However, it is generally impossible to determine the individual user or pharmacy from the exchanged information and therefore, security can be improved.

In addition, in the information processing system, information allowing to determine the individual user is not directly exchanged between apparatuses connected via the communication network 16. Therefore, there is no need to install an expensive apparatus whose security level is high such as IP-VPN in each apparatus such as the pharmacy internal system 11 and therefore, the cost can also be reduced.

In addition, information capable of directly determining each user or the institution such as a pharmacy is not recorded in the data server 111 in which user drug history information of many users is managed and therefore, even if information managed by the data server 111 is leaked, the damage of information leakage can be minimized. Further, information that directly determines the user or the like is not managed by the data server 111 and therefore, the cost of information management can be curbed.

Also in the information processing system, it is necessary for the user to hold the mobile terminal apparatus 15 over the identification information reader 31 or the like when drug history data is referred to and therefore, drug history data recorded in the data server 111 is not referred to from the pharmacy side or hospital side without permission of the user. Conversely, when the user or the pharmacist or the like refers to drug history data, the pharmacy side can make the user display personal information such as the date of birth so that the identity of the user can easily be confirmed. Accordingly, spoofing by others can be prevented.

In the foregoing, the exchange of data between the pharmacy internal system 11 and the data center 13 has been described, but a process similar to the above process described with reference to FIGS. 4 to 6 is performed for the exchange of data between the hospital internal system 12 and the data center 13.

In addition to pharmacies and hospitals, other third institutions such as insurance companies can read user's drug history data from the data server 111 or update drug history data by a similar process. That is, the information processing system shown in FIG. 1 can be used. In such a case, an ID uniquely identifying the third institution may be set up as an ID corresponding to the dispensing pharmacy ID and a third institution internal user ID in the third institution may be set up as an ID corresponding to the dispensing pharmacy internal user ID for each user.

Further, in the information processing system in FIG. 1, the user may be enabled to directly access the data server 111 without going through a pharmacy or hospital. In such a case, for example, the telephone number of the mobile phone as the mobile terminal apparatus 15 possessed by the user may be used as an ID corresponding to the dispensing pharmacy ID and the password known only to the user may be used as an ID corresponding to the dispensing pharmacy internal user ID. As the personal identification ID, the personal identification ID recorded in the mobile terminal apparatus 15 may be used.

Further in the foregoing, a case when data shared in the data server 111 is health data of users is taken as an example, but data to be shard may be any kind of data handled and shared by each local system.

The series of processes described above can be executed by hardware but can also be executed by software. When the series of processes is executed by software, a program that constructs such software is installed into a computer. Here, the expression "computer" includes a computer in which dedicated hardware is incorporated and a general-purpose personal computer or the like that is capable of executing various functions when various programs are installed.

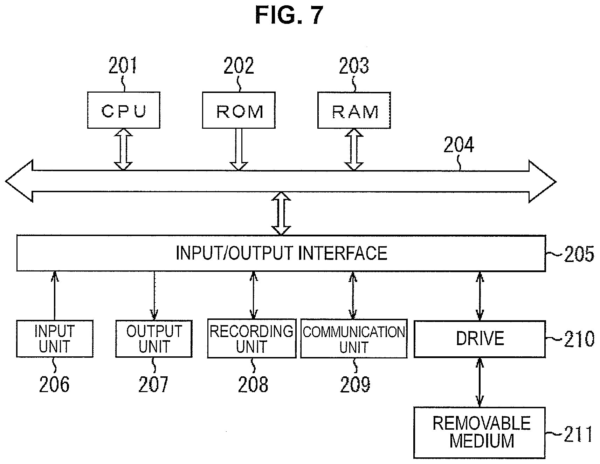

FIG. 7 is a block diagram showing a hardware configuration example of a computer that performs the above-described series of processing using a program.

In the computer, a central processing unit (CPU) 201, a read only memory (ROM) 202 and a random access memory (RAM) 203 are mutually connected by a bus 204.

An input/output interface 205 is also connected to the bus 204. An input unit 206, an output unit 207, a recording unit 208, a communication unit 209, and a drive 210 are connected to the input/output interface 205.

The input unit 206 is configured from a keyboard, a mouse, a microphone or the like. The output unit 207 is configured from a display, a speaker or the like. The recording unit 208 is configured from a hard disk, a non-volatile memory or the like. The communication unit 209 is configured from a network interface or the like. The drive 210 drives a removable medium 211 such as a magnetic disk, an optical disk, a magneto-optical disk, a semiconductor memory or the like.

In the computer configured as described above, the CPU 201 loads a program that is stored, for example, in the recording unit 208 onto the RAM 203 via the input/output interface 205 and the bus 204, and executes the program. Thus, the above-described series of processing is performed.

Programs to be executed by the computer (the CPU 201) are provided being recorded in the removable medium 211 which is a packaged medium or the like. Also, programs may be provided via a wired or wireless transmission medium, such as a local area network, the Internet or digital satellite broadcasting.

Then, by inserting the removable medium 211 into the drive 210, the program can be installed in the recording unit 208 via the input/output interface 205. Further, the program can be received by the communication unit 209 via a wired or wireless transmission medium and installed in the recording unit 208. Moreover, the program can be installed in advance in the ROM 202 or the recording unit 208.

It should be noted that the program executed by a computer may be a program that is processed in time series according to the sequence described in this specification or a program that is processed in parallel or at necessary timing such as upon calling.

For example, the present technology may also be configured as cloud computing that allocates or shares a function among a plurality of apparatuses via a network so as to perform processing.

Further, each step described by the above mentioned flow charts can be executed by one apparatus or by allocating a plurality of apparatuses.

In addition, in the case where a plurality of processes is included in one step, the plurality of processes included in this one step can be executed by one apparatus or by allocating a plurality of apparatuses.

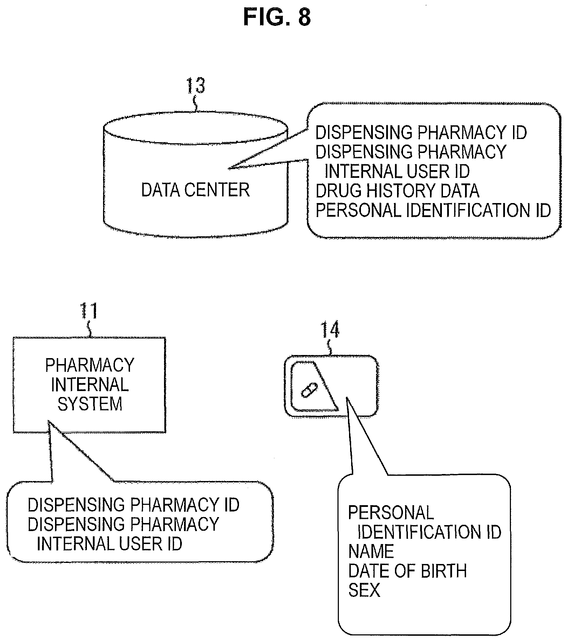

In the foregoing, it is assumed that personal information such as the name, date of birth, address, telephone number, insurance card No. and the like of the user is recorded in the pharmacy internal database 51 or the hospital internal database 91, but such personal information may also be recorded in, for example, as shown in FIG. 8, the IC card 14. In FIG. 8, the same reference signs are attached to elements corresponding to those in FIG. 1 and the description thereof is omitted when appropriate.

In the example of FIG. 8, the personal identification ID specific to the user (patient) and personal information of the user such as the name, date of birth, sex and the like are recorded in the IC card 14 possessed by the user. In addition, personal information recorded in the IC card 14 may further include the address, telephone number, insurance card No. of the user and the like.

When the personal identification ID and personal information of the user are recorded in the IC card 14 as described above, personal information of the user is not recorded in the pharmacy internal system 11, more specifically, in the pharmacy internal database 51 of the pharmacy internal medical receipt computer 33.

In this example, the dispending pharmacy ID or dispending pharmacy internal user ID is recorded in the pharmacy internal system 11 as information about the user or the like and personal information of the user is not recorded. In addition, drug history data may be recorded in the pharmacy internal system 11.

Like the pharmacy internal system 11, the hospital ID or hospital internal user ID is recorded in the hospital internal system 12 without personal information of the user being recorded.

Further in this case, like in the case of FIG. 1, the dispensing pharmacy ID (hospital ID), dispensing pharmacy internal user ID (hospital internal user ID), drug history data, personal identification ID and the like are recorded in the data center 13, more specifically, in the server database 121 of the data server 111.

When the personal identification ID and personal information of the user are recorded in the IC card 14 as described above, the personal information is read from the IC card 14 and used by the pharmacy internal system 11 or the hospital internal system 12 when necessary in each process described with reference to FIGS. 4 and 6.

In this case, personal information of the user is recorded in the IC card 14 and the personal information is not recorded in the pharmacy internal system 11 or the hospital internal system 12 and so if the IC card 14 is once issued (or registered), registration work in other pharmacies is not needed. That is, there is no need to input personal information and the like into the pharmacy internal system 11 in a pharmacy or the like used for the first time. Then, the identity of the user can be confirmed by reading personal information from the IC card 14 when necessary in the pharmacy internal system 11 or the like.

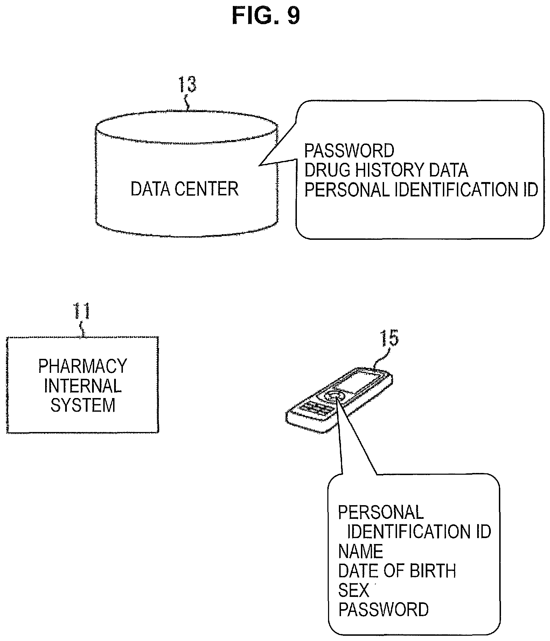

Further, as shown in FIG. 9, the personal identification ID and personal information such as the name, date of birth, sex and the like of the user may be recorded in the mobile terminal apparatus 15 such as a mobile phone possessed by the user. In addition, personal information recorded in the mobile terminal apparatus 15 may further include the address, telephone number, insurance card No. of the user and the like. In FIG. 9, the same reference signs are attached to elements corresponding to those in FIG. 1 and the description thereof is omitted when appropriate.

In the example of FIG. 9, like in FIG. 8, personal information of the user is not recorded in the pharmacy internal system 11 or the hospital internal system 12 and the personal information is read from the mobile terminal apparatus 15 and used when necessary in the pharmacy internal system 11 or the hospital internal system 12.

Further in the example of FIG. 9, the password preset by the user or the like is recorded in the mobile terminal apparatus 15 and the password is also recorded in the data center 13, in addition to drug history data and the personal identification ID. The password is used for personal authentication when a drug history is referred to.

That is, the user can refer to the drug history recorded in the data center 13 by using the mobile terminal apparatus 15. In this case, the mobile terminal apparatus 15 transmits the personal identification ID and password to the data center 13 in accordance with user's operation.

Then, the data center 13 reads drug history data associated with the personal identification ID and password received from the mobile terminal apparatus 15 and transmits the drug history data to the mobile terminal apparatus 15. The mobile terminal apparatus 15 receives the drug history data transmitted from the data center 13 and displays the data when appropriate.

By recording the personal identification ID and password in the data center 13 and the mobile terminal apparatus 15 as described above, the user can refer to the user's drug history data by operating the mobile terminal apparatus 15 without visiting a pharmacy.

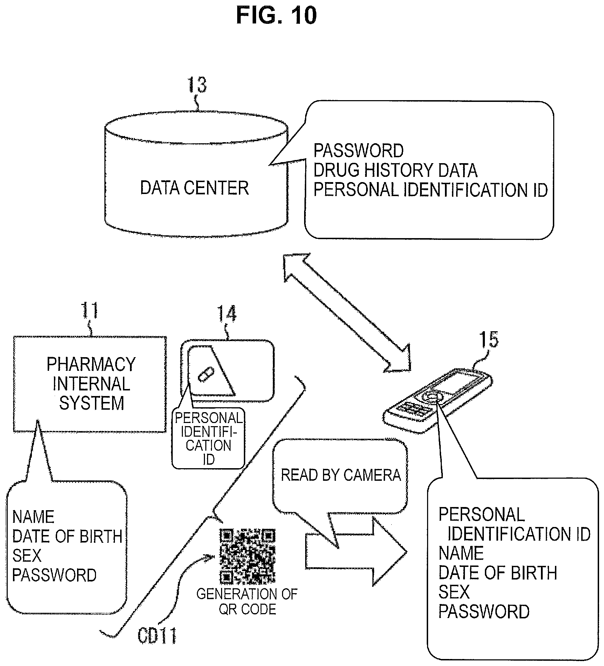

Further, as shown in FIG. 10, the mobile terminal apparatus 15 may read and record personal information such as the name, date of birth, sex and the like of the user, the personal identification ID, and the password from a QR code (registered trademark) CD11. In FIG. 10, the same reference signs are attached to elements corresponding to those in FIG. 1 and the description thereof is omitted when appropriate.

In the example of FIG. 10, the personal identification ID is recorded in the IC card 14 of the user and personal information such as the name, date of birth, sex and the like of the user and the password are recorded in the pharmacy internal system 11. In the data center 13, in addition to drug history information and the personal identification ID, the password is recorded.

In such a case, personal information such as the name, date of birth, sex and the like, the personal identification ID, and the password are not recorded in the mobile terminal apparatus 15 in its initial state.

When, for example, the user possesses the IC card 14 and the mobile terminal apparatus 15 and uses a pharmacy provided with the pharmacy internal system 11, the pharmacist or the like operates the pharmacy internal system 11 in accordance with a user's desire to generate the QR code (registered trademark) CD11. That is, the pharmacy internal system 11 reads the personal identification ID from the IC card 14 and also generates the QR code (registered trademark) CD11 from the read personal identification ID, personal information such as the name, date of birth, sex, and the like of the recorded user, and the password.

The QR code (registered trademark) CD11 contains personal information such as the name, date of birth, sex and the like of the user, the password, and the personal identification ID as information.

Then, the user causes the mobile terminal apparatus 15 to read and record information contained in the QR code (registered trademark) CD11 by using a camera or the like provided in the mobile terminal apparatus 15. Accordingly, personal information such as the name, date of birth, sex and the like of the user, the password, and the personal identification ID read from the QR code (registered trademark) CD11 are recorded in the mobile terminal apparatus 15.

Then, the user can subsequently refer to drug history data and the like by operating the mobile terminal apparatus 15 to, like the example in FIG. 9, directly access the data center 13 from the mobile terminal apparatus 15.

### Reference Signs List

- 11: pharmacy internal system
- 13: data center
- 32: pharmacy internal apparatus
- 33: pharmacy internal medical receipt computer
- 41: acquisition unit
- 42: data controller
- 43: communication unit
- 51: pharmacy internal database
- 111: data server
- 121: server internal database
- 122: communication unit
- 123: update unit
- 124: user search unit
- 125: drug history search unit

## Claims

1. An information processing apparatus comprising:
a receiving unit (122) adapted to receive personal identification information corresponding to a user for which personal information identifying the user is stored in a local system and system identification information to identify the local system, wherein the local system is configured to store personal information for a plurality of users and to associate the personal information identifying the user with the user corresponding to the personal identification information based on system internal user identification information in order to identify the user;
a recording unit (121) adapted to associate and record the personal identification information, the system identification information, the system internal user identification information used in the local system to identify the user, and shared data about the user, wherein the shared data is data on health of the user that is shared by the local system and the information processing apparatus;
a data search unit (125) adapted to search for the shared data associated with the received personal identification information;
a user search unit (124) adapted to search for the system internal user identification information associated with the received personal identification information and the received system identification information; and
a transmitting unit (122) adapted to transmit the shared data and the system internal user identification information that are obtained in a search.

2. The information processing apparatus according to claim 1,
wherein the receiving unit further includes an update unit adapted to receive the personal identification information, the system identification information, the system internal user identification information, and new shared data and associate the personal identification information, the system identification information, the system internal user identification information, and the new shared data that are received, to record the information and the data in the recording unit.

3. The information processing apparatus according to claim 2,
wherein the system identification information is a hash value obtained by hashing information determining the local system.

4. An information processing method of an information processing apparatus, the method comprising:
receiving personal identification information corresponding to a user for which personal information identifying the user is stored in a local system and system identification information to identify the local system, wherein the local system stores personal information for a plurality of users and associates the personal information identifying the user with the user corresponding to the personal identification information based on system internal user identification information in order to identify the user;
recording the received personal identification information corresponding to the user, the received system identification information to identify the local system, the system internal user identification information used in the local system to identify the user and shared data, wherein the shared data is data on health of the user that is shared by the local system and the information processing apparatus;
searching for shared data associated with the received personal identification information ;
searching for the system internal user identification information associated with the received personal identification information and the received system identification information ; and
transmitting the shared data and the system internal user identification information that are obtained in the search.

5. A program for causing a computer that controls an information processing apparatus to execute the method of claim 4.

6. An information processing apparatus comprising:
an acquisition unit (41, 81) adapted to acquire personal identification information corresponding to a user for which personal information identifying the user is stored by a local system including the information processing apparatus;
a transmitting unit (43, 83) adapted to transmit system identification information identifying the local system including the information processing apparatus and the personal identification information to another information processing apparatus (111) that is adapted to associate and record the personal identification information, the system identification information, system internal user identification information used in the local system to identify the user, and shared data about the user, wherein the shared data is data on health of the user that is shared by the local system and the other information processing apparatus; and
a receiving unit (43, 83) that is adapted to receive, from the other information processing apparatus, the shared data associated with the transmitted personal identification information and the system internal user identification information associated with the personal identification information and the system identification information that are transmitted, wherein the information processing apparatus is configured to store personal information for a plurality of users and to associate the personal information identifying the user with the user corresponding to the personal identification information based on the system internal user identification information in order to identify the user.

7. The information processing apparatus according to claim 6,
wherein the system identification information is a hash value obtained by hashing information determining the local system.

8. The information processing apparatus according to claim 7, wherein
the local system includes a recording unit adapted to associate and record the system identification information, the system internal user identification information, and the shared data.

9. An information processing method of an information processing apparatus, the method comprising:
acquiring personal identification information corresponding to a user for which personal information identifying the user is stored by a local system including the information processing apparatus;
transmitting system identification information identifying the local system including the information processing apparatus and the personal identification information to another information processing apparatus that associates and records the personal identification information, the system identification information, system internal user identification information used in the local system to identify the user, and shared data about the user, wherein the shared data is data on health of the user that is shared by the local system and the other information processing apparatus;
receiving, from the other information processing apparatus, the shared data associated with the transmitted personal identification information and the system internal user identification information associated with the personal identification information and the system identification information that are transmitted; and
storing personal information for a plurality of users and associating the personal information identifying the user with the user corresponding to the personal identification information based on the system internal user identification information in order to identify the user.

10. A program for causing a computer that controls an information processing apparatus to execute the method of claim 9.

## Patentansprüche

1. Informationsverarbeitungsvorrichtung, aufweisend:
eine Empfangseinheit (122), die ausgebildet ist, persönliche Identifizierungsinformationen, die einem Anwender entsprechen, für den persönliche Informationen, die den Anwender identifizieren, in einem örtlichen System gespeichert sind, und Systemidentifizierungsinformationen zum Identifizieren des örtlichen Systems zu empfangen, wobei das örtliche System konfiguriert ist, persönliche Informationen für eine Vielzahl von Anwendern zu speichern und die persönlichen Informationen, die den Anwender identifizieren, mit dem Anwender entsprechend den persönlichen Identifizierungsinformationen basierend auf systeminternen Anwenderidentifizierungsinformationen zu verknüpfen, um den Anwender zu identifizieren;
eine Aufzeichnungseinheit (121), die ausgebildet ist, die persönlichen Identifizierungsinformationen, die Systemidentifizierungsinformationen, die systeminternen Anwenderidentifizierungsinformationen, die in dem örtlichen System zum Identifizieren des Anwenders verwendet werden, und gemeinsame Daten über den Anwender zu verknüpfen und aufzuzeichnen, wobei die gemeinsamen Daten Daten über Gesundheit des Anwenders sind, die sich das örtliche System und die Informationsverarbeitungsvorrichtung teilen;
eine Datensucheinheit (125), die ausgebildet ist, nach den gemeinsamen Daten zu suchen, die mit den empfangenen persönlichen Identifizierungsinformationen verknüpft sind;
eine Anwendersucheinheit (124), die ausgebildet ist, nach den systeminternen Anwenderidentifizierungsinformationen in Verknüpfung mit den empfangenen persönlichen Identifizierungsinformationen und den empfangenen Systemidentifizierungsinformationen zu suchen; und
eine Sendeeinheit (122), die ausgebildet ist, die gemeinsamen Daten und die systeminternen Anwenderidentifizierungsinformationen, die in einer Suche erhalten werden, zu senden.

2. Informationsverarbeitungsvorrichtung nach Anspruch 1,
wobei die Empfangseinheit ferner eine Aktualisierungseinheit enthält, die ausgebildet ist, die persönlichen Identifizierungsinformationen, die Systemidentifizierungsinformationen, die systeminternen Anwenderidentifizierungsinformationen und neue gemeinsame Daten zu empfangen und die persönlichen Identifizierungsinformationen, die Systemidentifizierungsinformationen, die systeminternen Anwenderidentifizierungsinformationen und die neuen gemeinsamen Daten, die empfangen werden, zu verknüpfen, um die Informationen und die Daten in der Aufzeichnungseinheit aufzuzeichnen.

3. Informationsverarbeitungsvorrichtung nach Anspruch 2,
wobei die Systemidentifizierungsinformationen ein Hash-Wert sind, der durch Hashen von Informationen erhalten wird, die das örtliche System bestimmen.

4. Informationsverarbeitungsverfahren einer Informationsverarbeitungsvorrichtung, das Verfahren aufweisend:
Empfangen persönlicher Identifizierungsinformationen, die einem Anwender entsprechen, für den persönliche Informationen, die den Anwender identifizieren, in einem örtlichen System gespeichert sind, und von Systemidentifizierungsinformationen zum Identifizieren des örtlichen Systems, wobei das örtliche System persönliche Informationen für eine Vielzahl von Anwendern speichert und die persönlichen Informationen, die den Anwender identifizieren, mit dem Anwender entsprechend den persönlichen Identifizierungsinformationen basierend auf systeminternen Anwenderidentifizierungsinformationen verknüpft, um den Anwender zu identifizieren;
Aufzeichnen der empfangenen persönlichen Identifizierungsinformationen, die dem Anwender entsprechen, der empfangenen Systemidentifizierungsinformationen zum Identifizieren des örtlichen Systems, der systeminternen Anwenderidentifizierungsinformationen, die in dem örtlichen System zum Identifizieren des Anwenders verwendet werden, und gemeinsamer Daten, wobei die gemeinsamen Daten Daten über Gesundheit des Anwenders sind, die sich das örtliche System und die Informationsverarbeitungsvorrichtung teilen;
Suchen nach gemeinsamen Daten, die mit den empfangenen persönlichen Identifizierungsinformationen verknüpft sind;
Suchen nach den systeminternen Anwenderidentifizierungsinformationen verknüpft mit den empfangenen persönlichen Identifizierungsinformationen und den empfangenen Systemidentifizierungsinformationen; und
Senden der gemeinsamen Daten und der systeminternen Anwenderidentifizierungsinformationen, die in der Suche erhalten werden.

5. Programm, das einen Computer, der eine Informationsverarbeitungsvorrichtung steuert, veranlasst, das Verfahren nach Anspruch 4, auszuführen.

6. Informationsverarbeitungsvorrichtung, aufweisend:
eine Erlangungseinheit (41, 81), die ausgebildet ist, persönliche Identifizierungsinformationen, die einem Anwender entsprechen, für den persönliche Identifizierungsinformationen, die den Anwender identifizieren, durch ein örtliches System gespeichert sind, das die Informationsverarbeitungsvorrichtung enthält, zu erlangen;
eine Sendeeinheit (43, 83), die ausgebildet ist, Systemidentifizierungsinformationen, die das örtliche System identifizieren, das die Informationsverarbeitungsvorrichtung enthält, und die persönlichen Identifizierungsinformationen zu einer anderen Informationsverarbeitungsvorrichtung (111) zu senden, die ausgebildet ist, die persönlichen Identifizierungsinformationen, die Systemidentifizierungsinformationen, systeminterne Anwenderidentifizierungsinformationen, die in dem örtlichen System zum Identifizieren des Anwenders verwendet werden, und gemeinsame Daten über den Anwender zu verknüpfen und aufzuzeichnen, wobei die gemeinsamen Daten Daten über Gesundheit des Anwenders sind, die sich das örtliche System und die andere Informationsverarbeitungsvorrichtung teilen; und
eine Empfangseinheit (43, 83), die ausgebildet ist, von der anderen Informationsverarbeitungsvorrichtung die gemeinsamen Daten die mit den gesendeten persönlichen Identifizierungsinformationen verknüpft sind, und die systeminternen Anwenderidentifizierungsinformationen, die mit den persönlichen Identifizierungsinformationen verknüpft sind, und die Systemidentifizierungsinformationen, die gesendet werden, zu empfangen, wobei die Informationsverarbeitungsvorrichtung konfiguriert ist, persönliche Informationen für eine Vielzahl von Anwendern zu speichern und die persönlichen Informationen, die den Anwender identifizieren, mit dem Anwender entsprechend den persönlichen Identifizierungsinformationen basierend auf den systeminternen Anwenderidentifizierungsinformationen zu verknüpfen, um den Anwender zu identifizieren.

7. Informationsverarbeitungsvorrichtung nach Anspruch 6,
wobei die Systemidentifizierungsinformationen ein Hash-Wert sind, der durch Hashen von Informationen erhalten wird, die das örtliche System bestimmen.

8. Informationsverarbeitungsvorrichtung nach Anspruch 7, wobei
das örtliche System eine Aufzeichnungseinheit enthält, die ausgebildet ist, die Systemidentifizierungsinformationen, die systeminternen Anwenderidentifizierungsinformationen und die gemeinsamen Daten zu verknüpfen und aufzuzeichnen.

9. Informationsverarbeitungsverfahren einer Informationsverarbeitungsvorrichtung das Verfahren aufweisend:
Erlangen persönlicher Identifizierungsinformationen, die einem Anwender entsprechen, für den persönliche Informationen, die den Anwender identifizieren, durch ein örtliches System gespeichert sind, das die Informationsverarbeitungsvorrichtung enthält;
Senden von Systemidentifizierungsinformationen, die das örtliche System identifizieren, das die Informationsverarbeitungsvorrichtung enthält, und der persönlichen Identifizierungsinformationen zu einer anderen Informationsverarbeitungsvorrichtung, die die persönlichen Identifizierungsinformationen, die Systemidentifizierungsinformationen, systeminterne Anwenderidentifizierungsinformationen, die im örtlichen System zum Identifizieren des Anwenders verwendet werden, und gemeinsame Daten über den Anwender verknüpft und aufzeichnet, wobei die gemeinsamen Daten Daten über Gesundheit des Anwenders sind, die sich das örtliche System und die andere Informationsverarbeitungsvorrichtung teilen;
Empfangen, von der anderen Informationsverarbeitungsvorrichtung, der gemeinsamen Daten, die mit den gesendeten persönlichen Identifizierungsinformationen verknüpft sind, und der systeminternen Anwenderidentifizierungsinformationen, die mit den persönlichen Identifizierungsinformationen verknüpft sind, und der Systemidentifizierungsinformationen, die gesendet werden; und
Speichern persönlicher Informationen für eine Vielzahl von Anwendern und Verknüpfen der persönlichen Informationen, die den Anwender identifizieren, mit dem Anwender entsprechend den persönlichen Identifizierungsinformationen basierend auf den systeminternen Anwenderidentifizierungsinformationen, um den Anwender zu identifizieren.

10. Programm, das einen Computer, der eine Informationsverarbeitungsvorrichtung steuert, veranlasst, das Verfahren nach Anspruch 9 auszuführen.

## Revendications

1. Appareil de traitement d'informations comprenant :
une unité de réception (122) conçue pour recevoir des informations d'identification personnelles correspondant à un utilisateur pour lequel des informations personnelles identifiant l'utilisateur sont stockées dans un système local, et des informations d'identification du système pour identifier le système local, dans lequel le système local est configuré pour stocker des informations personnelles pour une pluralité d'utilisateurs et pour associer les informations personnelles identifiant l'utilisateur à l'utilisateur correspondant aux informations d'identification personnelles en se basant sur des informations d'identification d'utilisateur propres au système afin d'identifier l'utilisateur ;
une unité d'enregistrement (121) conçue pour associer et enregistrer les informations d'identification personnelles, les informations d'identification du système, les informations d'identification d'utilisateur propres au système utilisées dans le système local pour identifier l'utilisateur, et des données partagées concernant l'utilisateur, dans lequel les données partagées sont des données concernant la santé de l'utilisateur qui sont partagées par le système local et l'appareil de traitement d'informations ;
une unité de recherche de données (125) conçue pour rechercher les données partagées associées aux informations d'identification personnelles reçues ;
une unité de recherche d'utilisateur (124) conçue pour rechercher les informations d'identification d'utilisateur propres au système associées aux informations d'identification personnelles reçues et aux informations reçues d'identification du système ; et
une unité de transmission (122) conçue pour transmettre les données partagées et les informations d'identification d'utilisateur propres au système qui sont obtenues lors d'une recherche.

2. Appareil de traitement d'informations selon la revendication 1,
dans lequel l'unité de réception comprend en outre une unité de mise à jour conçue pour recevoir les informations d'identification personnelles, les informations d'identification du système, les informations d'identification d'utilisateur propres au système et de nouvelles données partagées et pour associer les informations d'identification personnelles, les informations d'identification du système, les informations d'identification d'utilisateur propres au système et les nouvelles données partagées qui sont reçues, pour enregistrer les informations et les données dans l'unité d'enregistrement.

3. Appareil de traitement d'informations selon la revendication 2,
dans lequel les informations d'identification du système constituent une valeur de hachage obtenue par hachage d'informations déterminant le système local.

4. Procédé de traitement d'informations d'un appareil de traitement d'informations, le procédé consistant :
à recevoir des informations d'identification personnelles correspondant à un utilisateur pour lequel des informations personnelles identifiant l'utilisateur sont stockées dans un système local, et des informations d'identification du système pour identifier le système local, dans lequel le système local stocke des informations personnelles pour une pluralité d'utilisateurs et associe les informations personnelles identifiant l'utilisateur à l'utilisateur correspondant aux informations d'identification personnelles en se basant sur des informations d'identification d'utilisateur propres au système afin d'identifier l'utilisateur ;
à enregistrer les informations d'identification personnelles reçues correspondant à l'utilisateur, les informations d'identification du système reçues pour identifier le système local, les informations d'identification d'utilisateur propres au système utilisées dans le système local pour identifier l'utilisateur, et des données partagées, dans lequel les données partagées sont des données concernant la santé de l'utilisateur qui sont partagées par le système local et l'appareil de traitement d'informations ;
à rechercher des données partagées associées aux informations d'identification personnelles reçues ;
à rechercher les informations d'identification d'utilisateur propres au système associées aux informations d'identification personnelles reçues et aux informations reçues d'identification du système ; et
à transmettre les données partagées et les informations d'identification d'utilisateur propres au système qui sont obtenues lors de la recherche.

5. Programme pour contraindre un ordinateur qui commande un appareil de traitement d'informations à exécuter le procédé selon la revendication 4.

6. Appareil de traitement d'informations comprenant :
une unité d'acquisition (41, 81) conçue pour acquérir des informations d'identification personnelles correspondant à un utilisateur pour lequel des informations personnelles identifiant l'utilisateur sont stockées par un système local comprenant l'appareil de traitement d'informations ;
une unité de transmission (43, 83) conçue pour transmettre des informations d'identification du système identifiant le système local comprenant l'appareil de traitement d'informations et les informations d'identification personnelles à un autre appareil de traitement d'informations (111) qui est conçu pour associer et enregistrer les informations d'identification personnelles, les informations d'identification du système, les informations d'identification d'utilisateur propres au système utilisées dans le système local pour identifier l'utilisateur, et des données partagées concernant l'utilisateur, dans lequel les données partagées sont des données concernant la santé de l'utilisateur qui sont partagées par le système local et l'autre appareil de traitement d'informations ; et
une unité de réception (43, 83) qui est conçue pour recevoir, de l'autre appareil de traitement d'informations, les données partagées associées aux informations d'identification personnelles transmises et les informations d'identification d'utilisateur propres au système associées aux informations d'identification personnelles et les informations d'identification du système qui sont transmises, l'appareil de traitement d'informations étant configuré pour stocker des informations personnelles pour une pluralité d'utilisateurs et pour associer les informations personnelles identifiant l'utilisateur à l'utilisateur correspondant aux informations d'identification personnelles en se basant sur les informations d'identification d'utilisateur propres au système afin d'identifier l'utilisateur.

7. Appareil de traitement d'informations selon la revendication 6,
dans lequel les informations d'identification du système constituent une valeur de hachage obtenue par hachage d'informations déterminant le système local.

8. Appareil de traitement d'informations selon la revendication 7, dans lequel
le système local comprend une unité d'enregistrement conçue pour associer et enregistrer les informations d'identification du système, les informations d'identification d'utilisateur propres au système et les données partagées.

9. Procédé de traitement d'informations d'un appareil de traitement d'informations, le procédé consistant :
à acquérir des informations d'identification personnelles correspondant à un utilisateur pour lequel des informations personnelles identifiant l'utilisateur sont stockées par un système local comprenant l'appareil de traitement d'informations ;
à transmettre des informations d'identification du système identifiant le système local comprenant l'appareil de traitement d'informations et les informations d'identification personnelles à un autre appareil de traitement d'informations qui associe et enregistre les informations d'identification personnelles, les informations d'identification du système, les informations d'identification d'utilisateur propres au système utilisées dans le système local pour identifier l'utilisateur, et des données partagées concernant l'utilisateur, dans lequel les données partagées sont des données concernant la santé de l'utilisateur qui sont partagées par le système local et l'autre appareil de traitement d'informations ; et
à recevoir, de l'autre appareil de traitement d'informations, les données partagées associées aux informations d'identification personnelles transmises et les informations d'identification d'utilisateur propres au système associées aux informations d'identification personnelles et les informations d'identification du système qui sont transmises ; et
à stocker des informations personnelles pour une pluralité d'utilisateurs et à associer les informations personnelles identifiant l'utilisateur à l'utilisateur correspondant aux informations d'identification personnelles en se basant sur les informations d'identification d'utilisateur propres au système afin d'identifier l'utilisateur.

10. Programme pour contraindre un ordinateur qui commande un appareil de traitement d'informations à exécuter le procédé selon la revendication 9.
